(19) Europäisches Patentamt European Patent Office Office européen des brevets

(11) **EP 3 631 018 B1**

(12) **EUROPEAN PATENT SPECIFICATION**

(45) Date of publication and mention
of the grant of the patent:
**24.07.2024 Bulletin 2024/30**

(21) Application number: **18732543.6**

(22) Date of filing: **25.05.2018**

(51) International Patent Classification (IPC):
***C12Q 1/6886*** *(2018.01)*    ***G16B 20/10*** *(2019.01)*
***G16B 30/10*** *(2019.01)*

(52) Cooperative Patent Classification (CPC):
(C-Sets available)
**C12Q 1/6886; G16B 20/10; G16B 30/10;**
C12Q 1/6869; G16B 40/00          (Cont.)

(86) International application number:
**PCT/US2018/034564**

(87) International publication number:
**WO 2018/218103 (29.11.2018 Gazette 2018/48)**

(54) **METHODS TO DETECT LARGE REARRANGEMENTS IN BRCA1/2**

VERFAHREN ZUR ERKENNUNG UMFANGREICHER NEUANORDNUNGEN BEI BRCA1/2

PROCÉDÉS POUR DÉTECTER DE GRANDS RÉARRANGEMENTS DANS BRCA1/2

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.05.2017 US 201762511815 P
12.06.2017 US 201762518383 P**

(43) Date of publication of application:
**08.04.2020 Bulletin 2020/15**

(73) Proprietor: **Life Technologies Corporation
Carlsbad, CA 92008 (US)**

(72) Inventors:
• **SCAFE, Charles**
  **Carlsbad, California 92008 (US)**
• **BRINZA, Dumitru**
  **Montara, California 94037 (US)**
• **VEITCH, James**
  **Carlsbad, California 92008 (US)**
• **QI, Rongsu**
  **Oviedo, FL 32765 (US)**
• **HYLAND, Fiona**
  **Carlsbad, California 92008 (US)**

(74) Representative: **Ziermann, Oliver
Life Technologies GmbH
Frankfurter Straße 129b
64293 Darmstadt (DE)**

(56) References cited:
• **ANONYMUS: "Ion AmpliSeq BRCA1 and BRCA2
  Panel", 2016, XP055747766, Retrieved from the
  Internet
  <URL:http://tools.thermofisher.com/content/sfs/
  brochures/BRCA12_Panel_Flyer.pdf> [retrieved
  on 20201106], DOI:
  10.1158/1538-7445.AM2013-4222**
• **YOSUKE HIROTSU ET AL: "Simultaneous
  detection of genetic and copy number alterations
  in BRCA1/2 genes", ONCOTARGET, vol. 8, no. 70,
  29 December 2017 (2017-12-29), United States,
  pages 114463 - 114473, XP055748316, ISSN:
  1949-2553, DOI: 10.18632/oncotarget.22962**
• **THADDEUS JUDKINS ET AL: "Clinical
  significance of large rearrangements in BRCA1
  and BRCA2 : Large Rearrangements in BRCA1
  and BRCA2", CANCER., vol. 118, no. 21, 27 April
  2012 (2012-04-27), US, pages 5210 - 5216,
  XP055493449, ISSN: 0008-543X, DOI:
  10.1002/cncr.27556**

**(Cont. next page)**

- SHIN SAEAM ET AL: "Evaluation of an amplicon-based next-generation sequencing panel for detection of BRCA1 and BRCA2 genetic variants", BREAST CANCER RESEARCH AND TREATMENT, SPRINGER , NY, US, vol. 158, no. 3, 6 July 2016 (2016-07-06), pages 433 - 440, XP036016294, ISSN: 0167-6806, [retrieved on 20160706], DOI: 10.1007/S10549-016-3891-Z
- ZANELLA ISABELLA ET AL: "Evaluation of the Ion Torrent PGM sequencing workflow for the routine rapid detection of BRCA1 and BRCA2 germline mutations.", EXPERIMENTAL AND MOLECULAR PATHOLOGY 04 2017, vol. 102, no. 2, 2 March 2017 (2017-03-02), pages 314 - 320, XP055493866, ISSN: 1096-0945
- LAILA C. SCHENKEL ET AL: "Clinical Next-Generation Sequencing Pipeline Outperforms a Combined Approach Using Sanger Sequencing and Multiplex Ligation-Dependent Probe Amplification in Targeted Gene Panel Analysis", JOURNAL OF MOLECULAR DIAGNOSTICS,THE, vol. 18, no. 5, September 2016 (2016-09-01), US, pages 657 - 667, XP055493401, ISSN: 1525-1578, DOI: 10.1016/j.jmoldx.2016.04.002
- ANNA FOWLER ET AL: "Accurate clinical detection of exon copy number variants in a targeted NGS panel using DECoN", WELLCOME OPEN RESEARCH, vol. 1, 25 November 2016 (2016-11-25), pages 20, XP055423472, DOI: 10.12688/wellcomeopenres.10069.1
- CATHERINE GRASSO ET AL: "Assessing Copy Number Alterations in Targeted, Amplicon-Based Next-Generation Sequencing Data", JOURNAL OF MOLECULAR DIAGNOSTICS,THE, vol. 17, no. 1, January 2015 (2015-01-01), US, pages 53 - 63, XP055493873, ISSN: 1525-1578, DOI: 10.1016/j.jmoldx.2014.09.008
- JARUPON FAH SATHIRAPONGSASUTI ET AL: "Exome sequencing-based copy-number variation and loss of heterozygosity detection: ExomeCNV", BIOINFORMATICS, vol. 27, no. 19, 9 August 2011 (2011-08-09), GB, pages 2648 - 2654, XP055465021, ISSN: 1367-4803, DOI: 10.1093/bioinformatics/btr462
- JIAN ZHANG ET AL: "Exome profiling of primary, metastatic and recurrent ovarian carcinomas in a BRCA1-positive patient", BMC CANCER, BIOMED CENTRAL, LONDON, GB, vol. 13, no. 1, 22 March 2013 (2013-03-22), pages 146, XP021146593, ISSN: 1471-2407, DOI: 10.1186/1471-2407-13-146
- FRANK REINECKE ET AL: "Quantitative analysis of differences in copy numbers using read depth obtained from PCR-enriched samples and controls", BMC BIOINFORMATICS, BIOMED CENTRAL, LONDON, GB, vol. 16, no. 1, 28 January 2015 (2015-01-28), pages 17, XP021220701, ISSN: 1471-2105, DOI: 10.1186/S12859-014-0428-5

(52) Cooperative Patent Classification (CPC): (Cont.)

C-Sets
C12Q 1/6869, C12Q 2537/165

**Description**

**BRIEF SUMMARY OF THE INVENTION**

Germline and somatic mutations in the BRCA1 and BRCA2 genes are involved in hereditary and non-hereditary breast and ovarian cancers. These genes are implicated in inherited risk and response to certain therapies. A test that detects these mutations from clinically relevant FFPE samples is valuable for both research and future clinical diagnostics purposes. Although small variants in these genes are commonly detected, large rearrangements such as exon level copy number variations are difficult to detect using traditional sequencing approaches. Large rearrangements represent a small, yet important portion of BRCA1/2 mutations, in addition to single nucleotide mutations and small insertion/deletions. The sizes of large rearrangements make them difficult to detect using traditional sequencing approaches, thereby requiring additional tests such as multiplex ligation dependent probe amplification (MLPA). There is a need for a next generation sequencing (NGS) assay with a comprehensive data analysis approach that is capable of detecting both small mutations and large rearrangements in a single assay with high sensitivity. There is a need for the NGS assay to be capable of detecting both small mutations and large rearrangements in formalin fixed paraffin embedded (FFPE) sample:

Hirotsu et al. 2017, Oncotarget 8(70):114463-114473, discloses the use of the Oncomine assay.

This need is addressed by the invention of claim 1. Advantageous embodiments are provided by the dependent claims.

According to an embodiment not claimed, but useful for understanding the invention, there is provided a kit comprising a set of primers associated with exons of BRCA1 and BRCA2 genes in a gene panel, the primers used in a method for detecting large rearrangements in the BRCA1 and BRCA2 genes, comprising: (a) amplifying a nucleic acid sample in the presence of a primer pool to produce a plurality of amplicons, the primer pool including a plurality of target specific primers targeting regions of exons of the BRCA1 and BRCA2 genes, wherein the target-specific primers for a region of an exon produce overlapping amplicons that cover the exon; (b) sequencing the amplicons to generate a plurality of reads; (c) mapping the reads to a reference sequence, wherein the reference sequence includes the BRCA1 and BRCA2 genes; (d) determining a number of reads per amplicon for the amplicons associated with the exons of the BRCA1 gene and a number of reads per amplicon for the amplicons associated with the exons of the BRCA2 gene; (e) determining exon copy numbers for the exons of the BRCA1 and BRCA2 genes based on the number of reads per amplicon for the amplicons associated with the exons of the BRCA1 and BRCA2 genes; (f) detecting an exon deletion or an exon duplication based on the exon copy numbers; and (g) detecting a whole gene deletion of BRCA1 gene or the BRCA2 gene based on the number of reads per amplicon for the amplicons associated with the exons of the BRCA1 and BRCA2 genes.

**BRIEF DESCRIPTION OF THE DRAWINGS**

The novel features of the invention are set forth in the appended claims. A better understanding of the features and advantages of the present invention will be obtained by reference to the following detailed description that sets forth illustrative embodiments, in which the principles of the invention are utilized, and the accompanying drawings of which:

FIG. 1 illustrates an example of using primer pairs to produce amplicons targeting an exon of BRCA1/2.

FIG. 2 illustrates an example of amplicons designed to cover an exon of BRCA1.

FIG. 3 is a block diagram of an exemplary method for detecting variants in BRCA1/2, in accordance with an embodiment.

FIG. 4 is a block diagram of an exemplary method for detecting large rearrangements, in accordance with an embodiment.

FIG. 5 shows exemplary results of amplicon sequence reads generated from five different data sets of FFPE DNA samples from prostate tumors.

FIG. 6 shows exemplary results of amplicon sequence reads sorted on GC content.

FIG. 7 shows an example of a box plot of normalized read counts per amplicon for exons of BRCA1/2 generated from a representative normal sample.

FIG. 8 shows an example of a box plot of normalized read counts per amplicon for exons of BRCA1/2, where the BRCA1 gene has exon deletions.

FIG. 9 shows an example of a box plot of normalized read counts per amplicon for exons of BRCA1/2, where the BRCA2 gene has exon duplications.

FIG. 10 shows an example of a box plot of normalized read counts per amplicon for exons of BRCA1/2, where the BRCA1 gene has an exon deletion.

FIG. 11 is a block diagram of an exemplary system for nucleic acid sequencing, in accordance with an embodiment.

**DETAILED DESCRIPTION OF THE INVENTION**

In various embodiments, DNA (deoxyribonucleic acid) may be referred to as a chain of nucleotides consisting of 4 types of nucleotides; A (adenine), T (thymine), C (cytosine), and G (guanine), and that RNA (ribonucleic acid) is comprised of 4 types of nucleotides; A, U (uracil), G, and C. Certain pairs of nucleotides specifically bind to one another in a complementary fashion (called complementary base pairing). That is, adenine (A) pairs with thymine (T) (in the case of RNA, however, adenine (A) pairs with uracil (U)), and cytosine (C) pairs with guanine (G). When a first nucleic acid strand binds to a second nucleic acid strand made up of nucleotides that are complementary to those in the first strand, the two strands bind to form a double strand. In various embodiments, "nucleic acid sequencing data," "nucleic acid sequencing information," "nucleic acid sequence," "genomic sequence," "genetic sequence," or "fragment sequence," or "nucleic acid sequencing read" denotes any information or data that is indicative of the order of the nucleotide bases (e.g., adenine, guanine, cytosine, and thymine/uracil) in a molecule (e.g., whole genome, whole transcriptome, exome, oligonucleotide, polynucleotide, fragment, etc.) of DNA or RNA.

In various embodiments, a "polynucleotide", "nucleic acid", or "oligonucleotide" refers to a linear polymer of nucleosides (including deoxyribonucleosides, ribonucleosides, or analogs thereof) joined by internucleosidic linkages. Typically, a polynucleotide comprises at least three nucleosides. Usually oligonucleotides range in size from a few monomeric units, e.g. 3-4, to several hundreds of monomeric units. Whenever a polynucleotide such as an oligonucleotide is represented by a sequence of letters, such as "ATGCCTG," it will be understood that the nucleotides are in 5'->3' order from left to right and that "A" denotes deoxyadenosine, "C" denotes deoxycytidine, "G" denotes deoxyguanosine, and "T" denotes thymidine, unless otherwise noted. The letters A, C, G, and T may be used to refer to the bases themselves, to nucleosides, or to nucleotides comprising the bases, as is standard in the art.

The phrase "next generation sequencing" or NGS refers to sequencing technologies having increased throughput as compared to traditional Sanger- and capillary electrophoresis-based approaches, for example with the ability to generate hundreds of thousands of relatively small sequence reads at a time. Some examples of next generation sequencing techniques include, but are not limited to, sequencing by synthesis, sequencing by ligation, and sequencing by hybridization.

As used herein, the terms "adapter" or "adapter and its complements" and their derivatives, refers to any linear oligonucleotide which can be ligated to a nucleic acid molecule of the disclosure. Optionally, the adapter includes a nucleic acid sequence that is not substantially complementary to the 3' end or the 5' end of at least one target sequences within the sample. In some embodiments, the adapter is substantially non-complementary to the 3' end or the 5' end of any target sequence present in the sample. In some embodiments, the adapter includes any single stranded or double-stranded linear oligonucleotide that is not substantially complementary to an amplified target sequence. In some embodiments, the adapter is substantially non-complementary to at least one, some or all of the nucleic acid molecules of the sample. In some embodiments, suitable adapter lengths are in the range of about 10-100 nucleotides, about 12-60 nucleotides and about 15-50 nucleotides in length. An adapter can include any combination of nucleotides and/or nucleic acids. In some aspects, the adapter can include one or more cleavable groups at one or more locations. In another aspect, the adapter can include a sequence that is substantially identical, or substantially complementary, to at least a portion of a primer, for example a universal primer. In some embodiments, the adapter can include a barcode or tag to assist with downstream cataloguing, identification or sequencing. In some embodiments, a single-stranded adapter can act as a substrate for amplification when ligated to an amplified target sequence, particularly in the presence of a polymerase and dNTPs under suitable temperature and pH.

As used herein, "DNA barcode" or "DNA tagging sequence" and its derivatives, refers to a unique short (e.g., 6-14 nucleotide) nucleic acid sequence within an adapter that can act as a 'key' to distinguish or separate a plurality of amplified target sequences in a sample. For the purposes of this disclosure, a DNA barcode or DNA tagging sequence can be incorporated into the nucleotide sequence of an adapter.

In various embodiments, target nucleic acids generated by the amplification of multiple target-specific sequences from a population of nucleic acid molecules can be sequenced. In some embodiments, the amplification can include hybridizing one or more target-specific primer pairs to the target sequence, extending a first primer of the primer pair, denaturing the extended first primer product from the population of nucleic acid molecules, hybridizing to the extended first primer product the second primer of the primer pair, extending the second primer to form a double stranded product, and digesting the target-specific primer pair away from the double stranded product to generate a plurality of amplified target sequences. In some embodiments, the amplified target sequences can be ligated to one or more adapters. In some embodiments, the adapters can include one or more nucleotide barcodes or tagging sequences. In some embodiments, the amplified target sequences once ligated to an adapter can undergo a nick translation reaction and/or further amplification to generate a library of adapter-ligated amplified target sequences. Exemplary methods of multiplex amplification are described in U.S. Patent Application Publication No. 2012/0295819, published November 22, 2012.

In various embodiments, the method of performing multiplex PCR amplification includes contacting a plurality of target-specific primer pairs having a forward and reverse primer, with a population of target sequences to form a plurality of template/primer duplexes; adding a DNA polymerase and a mixture of dNTPs to the plurality of template/primer duplexes for sufficient time and at sufficient temperature to extend either (or both) the forward or reverse primer in each target-specific primer pair via template-dependent synthesis thereby generating a plurality of extended primer product/template duplexes; denaturing the extended primer product/template duplexes; annealing to the extended primer product the complementary primer from the target-specific primer pair; and extending the annealed primer in the presence of a DNA polymerase and dNTPs to form a plurality of target-specific double-stranded nucleic acid molecules.

In some embodiments, the methods of the disclosure include selectively amplifying target sequences in a sample containing a plurality of nucleic acid molecules and ligating the amplified target sequences to at least one adapter and/or barcode. Adapters and barcodes for use in molecular biology library preparation techniques are well known to those of skill in the art. The definitions of adapters and barcodes as used herein are consistent with the terms used in the art. For example, the use of barcodes allows for the detection and analysis of multiple samples, sources, tissues or populations of nucleic acid molecules per multiplex reaction. A barcoded and amplified target sequence contains a unique nucleic acid sequence, typically a short 6-15 nucleotide sequence, that identifies and distinguishes one amplified nucleic acid molecule from another amplified nucleic acid molecule, even when both nucleic acid molecules minus the barcode contain the same nucleic acid sequence. The use of adapters allows for the amplification of each amplified nucleic acid molecule in a uniformed manner and helps reduce strand bias. Adapters can include universal adapters or propriety adapters both of which can be used downstream to perform one or more distinct functions. For example, amplified target sequences prepared by the methods disclosed herein can be ligated to an adapter that may be used downstream as a platform for clonal amplification. The adapter can function as a template strand for subsequent amplification using a second set of primers and therefore allows universal amplification of the adapter-ligated amplified target sequence. In some embodiments, selective amplification of target nucleic acids to generate a pool of amplicons can further comprise ligating one or more barcodes and/or adapters to an amplified target sequence. The ability to incorporate barcodes enhances sample throughput and allows for analysis of multiple samples or sources of material concurrently.

In this application, "reaction confinement region" generally refers to any region in which a reaction may be confined and includes, for example, a "reaction chamber," a "well," and a "microwell" (each of which may be used interchangeably). A reaction confinement region may include a region in which a physical or chemical attribute of a solid substrate can permit the localization of a reaction of interest, and a discrete region of a surface of a substrate that can specifically bind an analyte of interest (such as a discrete region with oligonucleotides or antibodies covalently linked to such surface), for example. Reaction confinement regions may be hollow or have well-defined shapes and volumes, which may be manufactured into a substrate. These latter types of reaction confinement regions are referred to herein as microwells or reaction chambers, and may be fabricated using any suitable microfabrication techniques. Reaction confinement regions may also be substantially flat areas on a substrate without wells, for example.

A plurality of defined spaces or reaction confinement regions may be arranged in an array, and each defined space or reaction confinement regions may be in electrical communication with at least one sensor to allow detection or measurement of one or more detectable or measurable parameter or characteristics. This array is referred to herein as a sensor array. The sensors may convert changes in the presence, concentration, or amounts of reaction by-products (or changes in ionic character of reactants) into an output signal, which may be registered electronically, for example, as a change in a voltage level or a current level which, in turn, may be processed to extract information about a chemical reaction or desired association event, for example, a nucleotide incorporation event. The sensors may include at least one chemically sensitive field effect transistor ("chemFET") that can be configured to generate at least one output signal related to a property of a chemical reaction or target analyte of interest in proximity thereof. Such properties can include a concentration (or a change in concentration) of a reactant, product or by-product, or a value of a physical property (or a change in such value), such as an ion concentration. An initial measurement or interrogation of a pH for a defined space or reaction confinement regions, for example, may be represented as an electrical signal or a voltage, which may be digitalized (e.g., converted to a digital representation of the electrical signal or the voltage). Any of these measurements and representations may be considered raw data or a raw signal.

As used herein, a "somatic variation" or "somatic mutation" can refer to a variation in genetic sequence that results from a mutation that occurs in a non-germline cell. The variation can be passed on to daughter cells through mitotic division. This can result in a group of cells having a genetic difference from the rest of the cells of an organism. Additionally, as the variation does not occur in a germline cell, the mutation may not be inherited by progeny organisms.

In some embodiments, the panel comprises the Oncomine BRCA Research NGS Assay available from Thermo Fisher Scientific (SKU A32840 or SKU A32841). The Oncomine BRCA Research NGS Assay covers 100% of all exons of BRCA1/2 with 265 amplicons (targeted regions) using primer pairs. The assay is compatible with DNA samples extracted from FFPE as well as blood samples and with automated and manual library preparation methods. The methods described herein detect exon level copy number variations, including large indels, exon/gene deletion/duplication events and small variants in BRCA1/2 using a single assay.

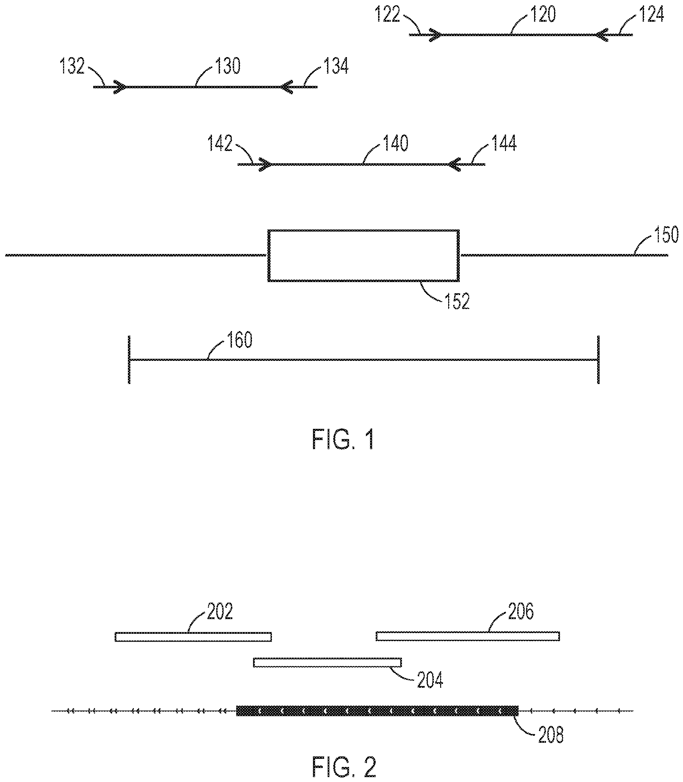

FIG. 1 illustrates an example of using primer pairs to produce amplicons targeting an exon of BRCA1/2. Amplicons 120, 130 and 140 partially overlap each other and together cover an exon 152 of the reference sequence 150, which includes the BRCA1 or BRCA2 gene. The primer pairs 122 and 124 for amplicon 120, primer pairs 132 and 134 for amplicon 130, and primer pairs 142 and 144 for amplicon 140, specifically target regions that overlap the exon 152. The range 160 is an example of the exon coverage region for the cluster of amplicons 120, 130 and 140. Amplification of the target regions of a nucleic acid sample corresponding to target specific primer pairs 122 and 124, 132 and 134, 142 and 144 can produce multiple copies of amplicons 120, 130 and 140, respectively. Amplification of the amplicons 120, 130 and 140 in the region of the exon 152 would produce a high density of amplicons, providing a sufficient volume of data for exon level copy number estimates. The example of a particular arrangement of the amplicons 120, 130 and 140 with respect to exon 152 is for illustrative purposes only and is not limiting.

FIG. 2 illustrates an example of amplicons designed to cover an exon of BRCA1. In this example, three amplicons 202, 204 and 206 are designed to span a region that includes an exon 208 of a BRCA1 reference sequence. This example is for illustrative purposes and is not limiting.

In some embodiments, a group of amplicons may cover an exon and regions adjacent to the exon. The number of amplicons in a group of amplicons may range from two to over 50. Typical numbers of amplicons in a group covering an exon is three to five. One or more amplicons in a group may not overlap the exon, however any one amplicon overlaps at least one other amplicon in the group. The group of amplicons together may cover the exon and regions adjacent to the exon.

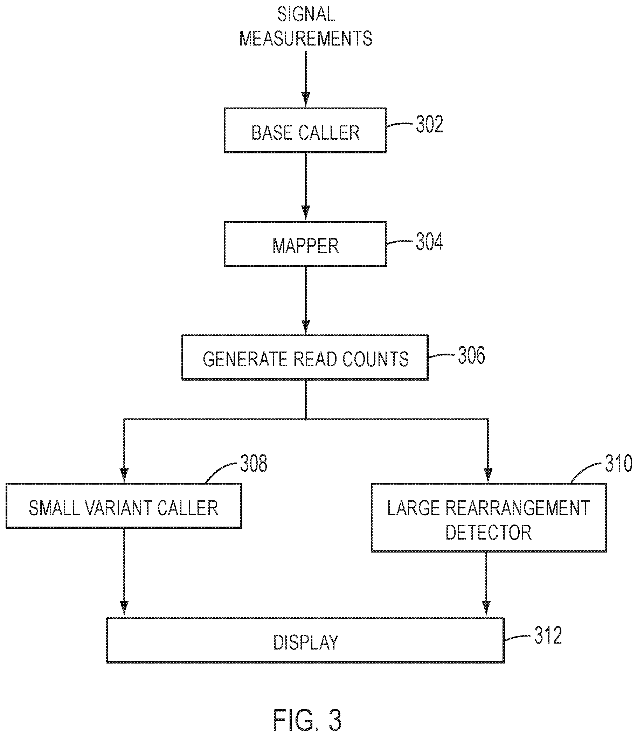

FIG. 3 is a block diagram of an exemplary method for detecting variants in BRCA1/2, in accordance with an embodiment. Signal measurements may be provided to a processor by a nucleic acid sequencing device. In some embodiments, each signal measurement represents a signal amplitude or intensity measured in response to an incorporation or non-incorporation of a flowed nucleotide by sample nucleic acids in microwells of a sensor array. For an incorporation event, the signal amplitudes depend on the number of bases incorporated at one flow. For homopolymers, the signal amplitudes increase with increasing homopolymer length. The processor may apply a base caller 302 to generate base calls for a sequence read by analyzing flow space signal measurements. The signal measurements may be raw acquisition data or data having been processed, such as, e.g., by scaling, background filtering, normalization, correction for signal decay, and/or correction for phase errors or effects, etc. The base calls may be made by analyzing any suitable signal characteristics (e.g., signal amplitude or intensity). The structure and/or design of a sensor array, signal processing and base calling for use with the present teachings may include one or more features described in U.S. Pat. Appl. Publ. No. 2013/0090860, April 11, 2013.

Once the base sequence for the sequence read is determined, the sequence reads may be provided to mapper 304. The mapper 304 aligns the sequence reads to a reference genome to determine aligned sequence reads and associated mapping quality parameters. Methods for aligning sequence reads for use with the present teachings may include one or more features described in U.S. Pat. Appl. Publ. No. 2012/0197623, published August 2, 2012. The aligned sequence reads may be provided for further processing, for example, in a BAM file.

The aligned sequence reads are associated with amplicons at specific locations relative to the reference genome. The read counts block 306 determines the number of reads per amplicon, referred to as coverage. The aligned sequence reads and the reads per amplicon may be provided to the small variant caller 308 and the large rearrangement detector 310.

The small variant caller 308 may detect small variants such as single nucleotide polymorphisms (SNP), insertion/deletions (indels) and multinucleotide polymorphisms (MNP). In some embodiments, the small variant detection methods for use with the present teachings may include one or more features described in U.S. Pat. Appl. Publ. No. 2013/0345066, published December 26, 2013, U.S. Pat. Appl. Publ. No. 2014/0296080, published October 2, 2014, and U.S. Pat. Appl. Publ. No. 2014/0052381, published February 20, 2014. In some embodiments, other variant detection methods may be used. In various embodiments, a variant caller can be configured to communicate variants called for a sample genome as a *.vcf, *.gff, or *.hdf data file. The called variant information can be communicated using any file format as long as the called variant information can be parsed and/or extracted for analysis.

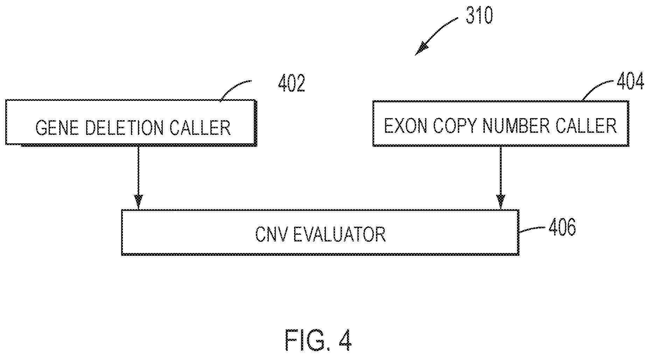

FIG. 4 is a block diagram of an exemplary method for detecting large rearrangements, in accordance with an embodiment. In some embodiments, the whole gene deletion caller 402 detects a gene deletion based on the number of reads per amplicon as follows:

    a. Divide the number of reads per amplicon for amplicons associated with exons of the BRCA1 gene by the total number of reads in the sample to form normalized read counts per amplicon associated with the BRCA1 gene.

    b. Calculate the mean and standard deviation of the normalized read counts per amplicon associated with the BRCA1 gene.

    c. Divide the number of reads per amplicon for amplicons associated with exons of the BRCA2 gene by the total number of reads in the sample to form normalized read counts per amplicon associated with the BRCA2 gene.

    d. Calculate the mean and standard deviation of the normalized read counts per amplicon associated with the BRCA2 gene.

    e. Apply a t-test based on the means and standard deviations calculated for the BRCA1 and BRCA2 genes to determine a test statistic and associated p-value.

    f. Compare the p-value to a first threshold to detect a whole gene deletion if the p-value is less than the first threshold. An exemplary value for first threshold value is $10^{-4}$, so that $p \leq 10^{-4}$. In some embodiments, the first threshold value may be set to a value in a range from $10^{-5}$ to $10^{-3}$.

    g. Calculate the PHRED score based on the p-value,

$$\text{PHRED score} = -10\log(\text{p-value})$$

The PHRED score of 40 corresponds to a p-value threshold of $10^{-4}$.

    h. Compare each gene's (standard deviation)/mean to a second threshold, if the (standard deviation)/mean is less than the second threshold. An exemplary value for the second threshold is 0.3, so that (standard deviation)/mean $\leq 0.3$. In some embodiments, the second threshold value may be set to a value in a range from 0.2 to 0.4.

    i. If the first and second threshold criteria are met, then the decision for a whole gene deletion event can be made.

In some embodiments, the whole gene deletion caller 402 may calculate statistics of the reads per amplicon associated with each exon. In particular, statistics of the normalized read counts per amplicon associated with the coding exons may be calculated. For example, a box plot representing statistics of normalized read counts per amplicon for each exon may include the range, upper and lower quartiles, and outliers for the exons of BRCA1 and BRCA2 in the sample. The box plot may be provided for display 312 to the user.

In some embodiments, the sample may include a sample identifier (ID) amplicons associated with a different chromosome than the BRCA1 (chromosome 17) and BRCA2 (chromosome 13). The base caller 302 and mapper 304 may process the sample ID amplicons along with the amplicons associated with the exons of BRCA1 and BRCA2. The whole gene deletion caller 402 may divide the number of sample ID reads by the total number of reads in the sample to form normalized sample ID read counts. The whole gene deletion caller 402 may calculate statistics of the normalized sample ID read counts. For example, a box plot element representing statistics of the normalized sample ID read counts may include the range, upper and lower quartiles, and outliers. The sample ID box plot element may be included with the box plot elements for the exons of BRCA1 and BRCA2 and provided for display 312 to the user.

In some embodiments, the large rearrangement detector 310 may include an exon copy number caller 404. The exon copy number caller 404 may compare the normalized read counts per amplicon to a baseline coverage for the associated exon to determine a candidate copy number for the exon. The baseline coverage can be created from a single control sample, however, in some embodiments the baseline coverage can be created by adding coverages from plurality of control samples and by adjusting the coverages by their known ploidy information. A median absolute pairwise difference (MAPD) can be calculated for the ratios of the normalized read counts per amplicon and the baseline coverage of adjacent amplicons for the exon. Since adjacent amplicons for a given exon should ideally have the same copy number, finding the median value of the absolute values of the differences of the copy number levels provides an indication of quality. The MAPD values for each exon may provide a quality for the candidate copy number for the exon. Methods for determining copy number variation for use with the present teachings may include one or more features described in U.S. Pat. Appl. Publ. No. 2014/0256571, published September 11, 2014. In some embodiments, methods for determining copy numbers on the gene level may be adapted to determine copy numbers on the exon level by using exon identifiers instead of gene identifiers.

The exon copy number caller 404 may apply a first scaling procedure to exon level candidate copy numbers for amplicons generated from a germline sample as follows:

a. Determine the highest value of the candidate copy numbers for exons of BRCA1 gene.

b. Determine the highest value of the candidate copy numbers for exons of the BRCA2 gene.

c. Compare the highest values for the BRCA1 gene and BRCA2 gene. Select the maximum candidate copy number and select the gene.

d. Define the upper limit on a range for the candidate copy numbers as the selected maximum candidate copy number.

e. Determine the median value of the candidate copy number values for exons of the selected gene having the selected maximum candidate copy number.

f. Set the median value to a reference level.

g. Scale the candidate copy numbers of the exons of both genes relative to the reference level.

The reference level may provide a convenient point of reference for copy number variations. For example, a reference level of two represents a diploid state of two copies of the exon or gene. The exon copy number caller 404 may apply a second scaling procedure to exon level candidate copy numbers for amplicons generated from a somatic sample as follows:

a. Determine the median value of the candidate copy numbers for the exons of BRCA1.

b. Set the median value to a reference level.

c. Scale the candidate copy numbers for the exons of BRCA1 relative to the reference level.

d. Determine the median value of the candidate copy numbers for the exons of BRCA2.

e. Set the median value to the reference level.

f. Scale the candidate copy numbers for the exons of BRCA2 relative to the reference level.

The exon copy number caller 404 may store the candidate copy numbers per exon and the corresponding MAPD values in one or more files.

The CNV evaluator 406 may receive files containing the candidate copy numbers for the exons, the corresponding MAPD values and the whole gene deletion information. In some embodiments, the CNV evaluator 406 may apply empirical rules to score the candidate copy numbers and assign each to one of four possible subtypes: exon deletion (BigDel), exon duplication (BigDup), reference (REF) and no call (NOCALL). The scoring may be based on MAPD values across exons and deviation of candidate copy numbers from integer values. The CNV evaluator 406 may merge copy number calls for adjacent exons if the candidate copy number values are within an interval of the same integer. Example ranges for the interval can be the integer value $\pm 0.3$, integer value $\pm 0.35$, integer value $\pm 0.2$, integer value $\pm 0.25$.

In some embodiments, the rules for combining copy number calls for adjacent exons may include one or more of the following:

a. Adjacent BigDel calls for exons are combined to form a merged BigDel call for a segment that includes two or more adjacent exons.

b. Adjacent BigDup calls for exons are combined to form a merged BigDup call for a segment that includes two or more adjacent exons.

c. Adjacent REF calls for exons are combined to form a merged REF call for a segment that includes two or more adjacent exons.

d. Adjacent NOCALLs for exons are combined to form a merged NOCALL for a segment that includes two or more adjacent exons.

e. BigDel adjacent to NOCALL adjacent to BigDel calls are combined to form a merged BigDel call for a segment that includes three adjacent exons.

f. BigDup adjacent to NOCALL adjacent to BigDup calls are combined to form a merged BigDup call for a segment that includes three adjacent exons.

The CNV evaluator 406 may provide the final version of copy number calls for the exons, merged calls for segments having two or more exons, and gene deletion information in an output file for display 312 to the user.

In some embodiments, graphical displays of normalized coverage counts for each exon in the panel can be used to confirm the calls and to suggest samples that may require further study. A display of normalized coverage of Sample ID amplicons can be used to calibrate copy-number gain or loss events of the BRCA1 and BRCA2 genes.

Table 1 shows a comparison of a previous BRCA1/2 research panel and the Oncomine™ BRCA Research Assay, which may be used with the methods described herein. Improvements include reducing the DNA required by half, compatibility with formalin fixed paraffin embedded (FFPE) samples and the ability to detect exon and gene deletions using methods described herein.

TABLE 1.

|  | Ion AmpliSeq™ BRCA1/2 Research Panel (Community Panel) | Oncomine™ BRCA Research Assay |
|---|---|---|
| Number of pools | 3 | 2 |
| DNA required | 20ng/per pool | 10 ng/pool or 10ng/total |
| Total Amplicons | 167 | 265 |
| Amplicons/pool | 55, 56, 56 | 132, 133 |
| Amplicon design (size) | Mostly 225 bp | Mostly <135 bp |
| Amplicon length range | 126 - 290 bp | 125 - 189 bp |
| Insert Overlap Minimum | 0b (adjacent) | 2b |
| Exon Padding Minimum | 6b | >15b (mean 34b) |
| Amplicon %GC | 24.6% - 56.6% | 26.9% - 56.2% |
| Sample ID | Not included | Included |
| Libraries per sample | 3 | 1 |
| FFPE compatible | No | Yes |
| Ion Chef™ compatible | No | Yes |
| Exon/Gene deletion detection | No | Yes |
| Manufacturing QC | Standard RUO | Enhanced |

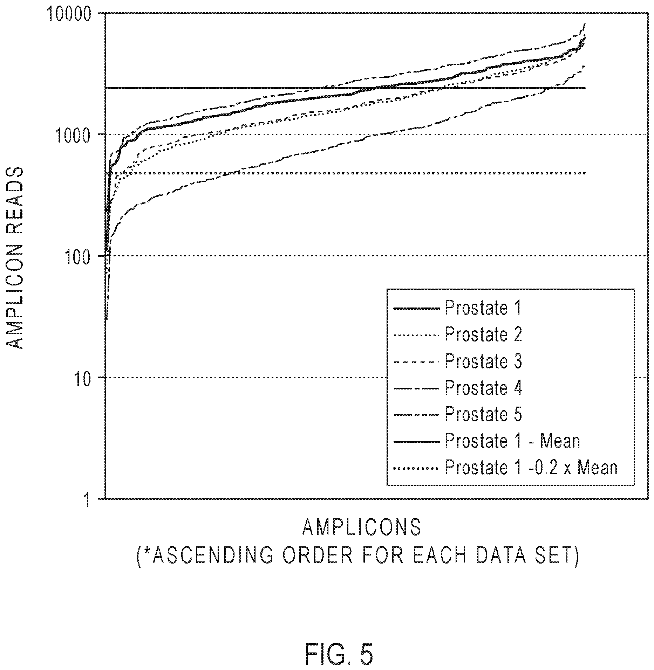

FIG. 5 shows exemplary results of amplicon sequence reads generated from five different data sets of FFPE DNA samples from prostate tumors. The x-axis is the set of amplicons sorted by the number of amplicon sequence reads in ascending order and the y-axis is the number of amplicon sequence reads. The results show that the BRCA1/2 amplicons are uniform and perform well with FFPE DNA. For the prostate 1 sample, the 0.2xMean line is shown. Uniformity can be indicated by the fraction of amplicon sequence reads that are greater than 0.2 times the mean number of amplicon sequence reads. The numbers of amplicon sequence reads for the prostate 1 sample are nearly all above the 0.2xMean line. Uniformity can be indicated by the fraction of amplicon sequence reads that are greater than 0.2 times the mean number of amplicon sequence reads.

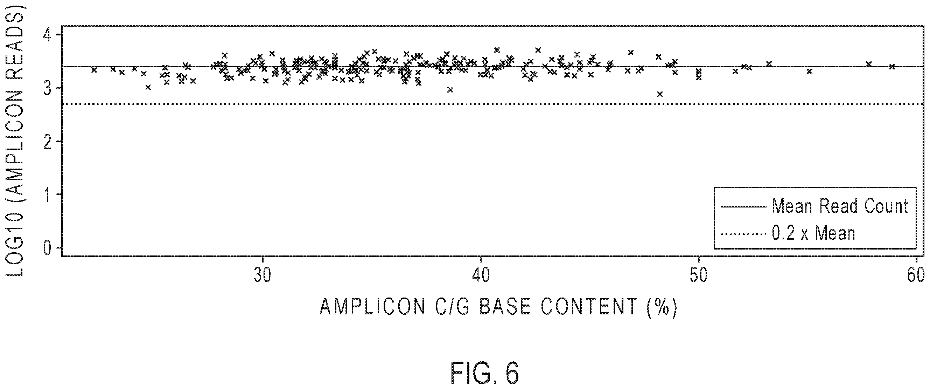

FIG. 6 shows exemplary results of amplicon sequence reads sorted on GC content. The x-axis gives the percent of G or C base content in the amplicon sequence reads and the y-axis gives the log 10 of the number of amplicon sequence reads. The results show uniformity, where all the values are above the 0.2xMean line.

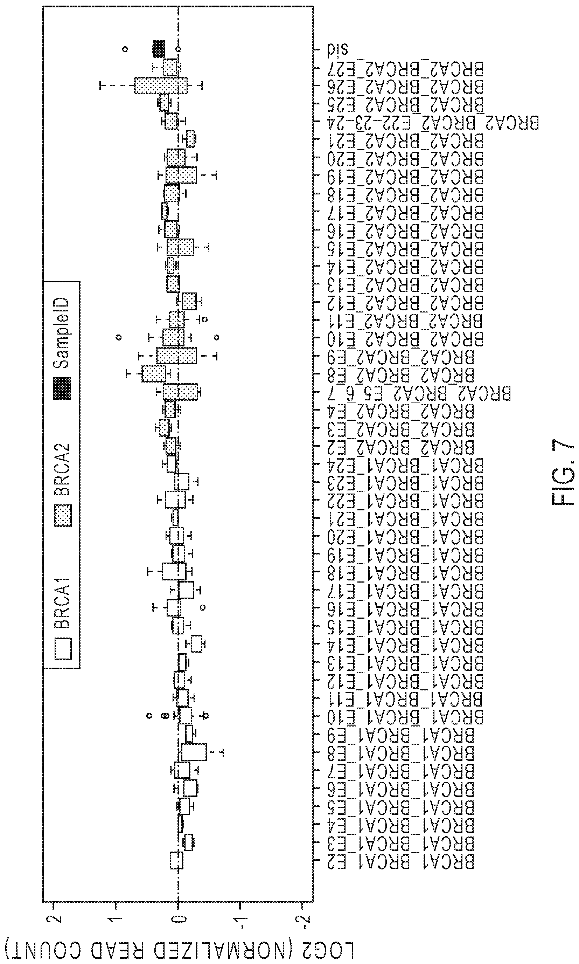

FIG. 7 shows an example of a box plot of normalized read counts per amplicon for exons of BRCA1/2 generated from a representative normal sample. The x-axis lists the exon designators and the y-axis gives the log2 of the normalized read counts. The y=0 level indicates a normal level. The box plots are generally close to the normal level. There may be some variation, which may be due to amplicon noise.

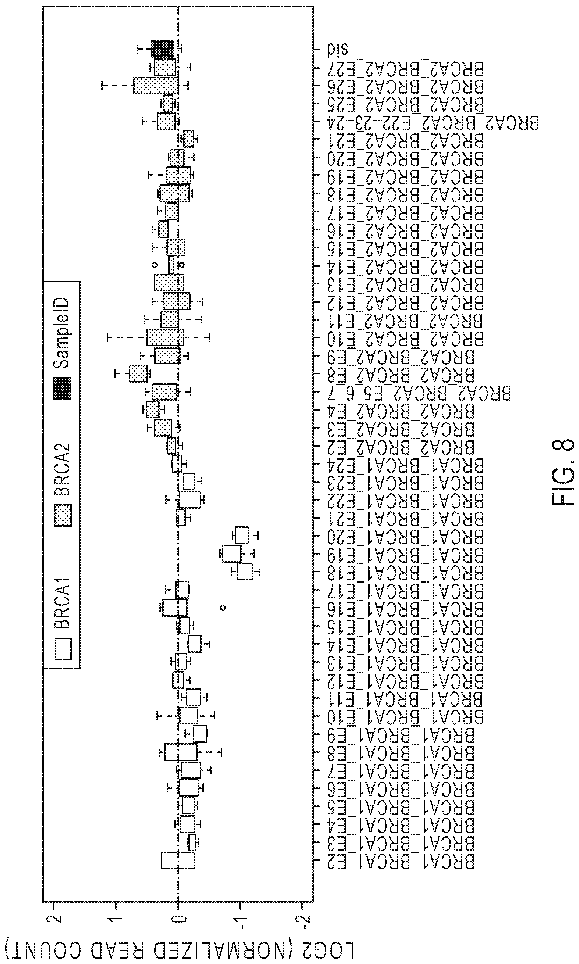

FIG. 8 shows an example of a box plot of normalized read counts per amplicon for exons of BRCA1/2, where the BRCA1 gene has exon deletions. The box plot shows exons 18-20 of the BRCA1 gene are clustered around a log2 value of -1, indicating half of the normal level of normalized read counts.

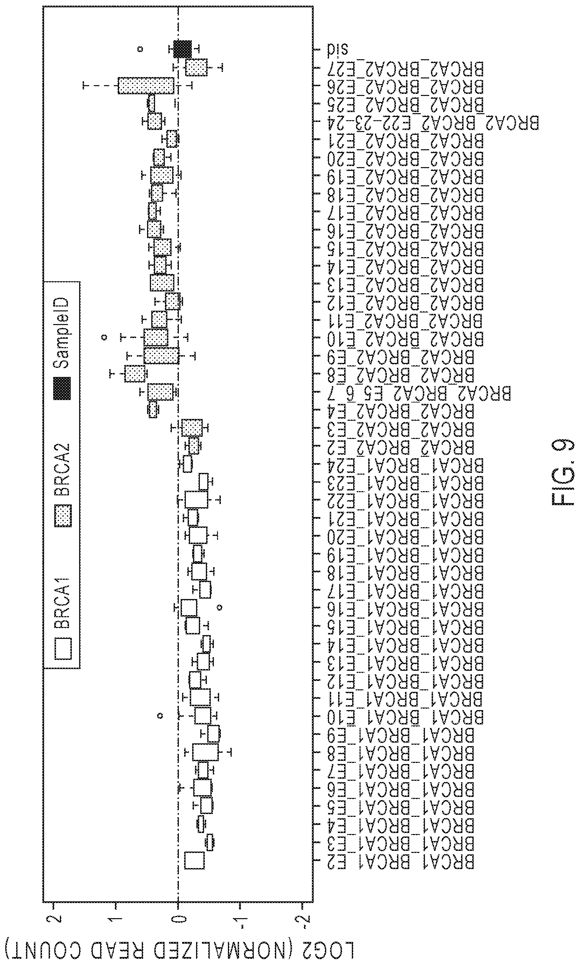

FIG. 9 shows an example of a box plot of normalized read counts per amplicon for exons of BRCA1/2, where the BRCA2 gene has exon duplications. The box plot shows exons 4-26 of the BRCA2 gene are clustered between 0 and 1, near log2(3/2), indicating 3/2 of the normal level of normalized read counts.

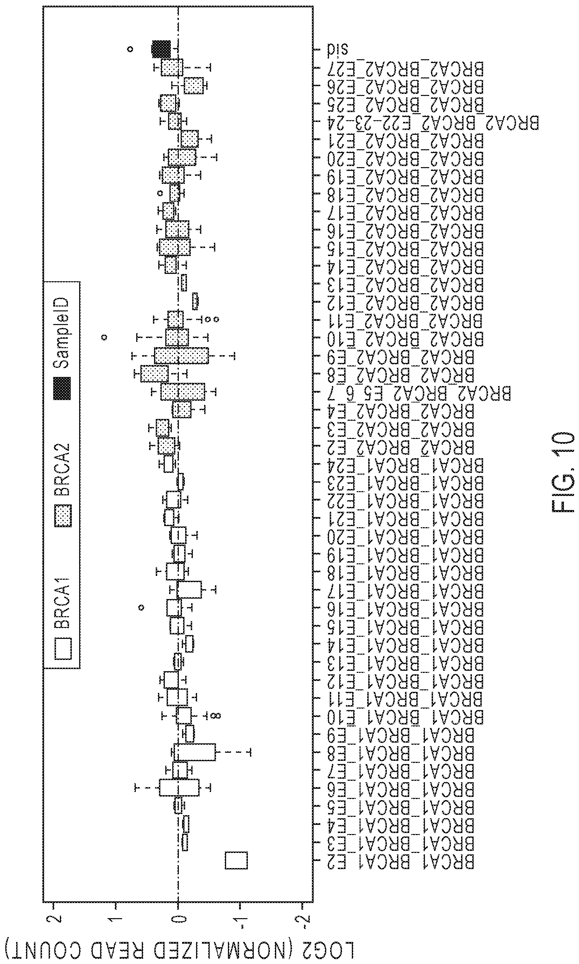

FIG. 10 shows an example of a box plot of normalized read counts per amplicon for exons of BRCA1/2, where the BRCA1 gene has an exon deletion. The box plot shows exon 2 of the BRCA1 gene is near a log2 value of -1, indicating half of the normal level of normalized read counts.

Table 2 gives a summary of exon deletion performance metrics for 193 samples tested and result compared with the known truth set.

TABLE 2.

| Sample Set | Sensitivity - Events | | Specificity - Events | | PPV - Events | | NoCallRate - Samples | |
|---|---|---|---|---|---|---|---|---|
| All Samples | 0.944 | | 0.949 | | 0.515 | | 0.367 | |
| | TP - Events | FP - Events | FN - Events | NoCall- Events | TN - Events | NC - Sample | TN - Sample | FP - Sample |
| All Samples | 17 | 16 | 1 | 177 | 201 | 71 | 90 | 14 |

In Table 2, TP is true positive, FP is false positive, FN is false negative, NC is no call, sensitivity is TP/(TP+FN), specificity is TN/(TN+FP) and positive predictive value PPV is TP/(TP+FP). The items NC - Sample, TN - Sample and FP - Sample indicate multiple events of the given type for a sample. The performance results show high sensitivity of 0.944 and high specificity of 0.949. The PPV value is affected by the merging of adjacent exons into a segment which is counted as a single unit. A false positive would have a very low chance of being merged with an adjacent exon. The effect of merging exons into larger segments for TPs and not for FPs reduces the ratio of TP to FP, leading to a lower PPV.

Table 3 gives results of a comparison of results large rearrangement detection (LRD) methods described herein with methods applying a Hidden Markov Model (HMM) and an Expectation Maximization (EM). The results for were compared to truth sets for the samples. The performance for large rearrangement detection (LRD) shows the highest number of true positives and the lowest number of false positives.

TABLE 3.

| Method | Runs | Samples | Truth | TP | FN | FP |
|---|---|---|---|---|---|---|
| EM | 10 | 203 | 22 | 20 | 0 | 45 |
| HMM | 11 | 219 | 23 | 11 | 12 | 20 |
| LRD | 11 | 219 | 23 | 22 | 1 | 18 |

According to an exemplary embodiment, there is provided a method for detecting large rearrangements in BRCA1 and BRCA2 genes, comprising: (a) amplifying a nucleic acid sample in the presence of a primer pool to produce a plurality of amplicons, the primer pool including a plurality of target specific primers targeting regions of exons of the BRCA1 and BRCA2 genes, wherein the target-specific primers for a region of an exon produce overlapping amplicons that cover the exon; (b) sequencing the amplicons to generate a plurality of reads; (c) mapping the reads to a reference sequence, wherein the reference sequence includes the BRCA1 and BRCA2 genes; (d) determining a number of reads per amplicon for the amplicons associated with the exons of the BRCA1 gene and a number of reads per amplicon for the amplicons associated with the exons of the BRCA2 gene; (e) determining exon copy numbers for the exons of the BRCA1 and BRCA2 genes based on the number of reads per amplicon for the amplicons associated with the exons of the BRCA1 and BRCA2 genes; (f) detecting an exon deletion or an exon duplication based on the exon copy numbers; and (g) detecting a whole gene deletion of BRCA1 gene or the BRCA2 gene based on the number of reads per amplicon for the amplicons associated with the exons of the BRCA1 and BRCA2 genes. The exons of the BRCA1 and BRCA2 genes may comprise coding exons. The method may further comprise dividing the number of reads per amplicon for amplicons associated with exons of the BRCA1 gene by a total number of reads of the amplicons generated from the nucleic acid sample to form normalized read counts per amplicon for the BRCA1 gene and dividing the number of reads per amplicon for amplicons associated with exons of the BRCA2 gene by a total number of reads of the amplicons generated from the nucleic acid sample to form normalized read counts per amplicon for the BRCA2 gene. The step of determining exon copy numbers may further comprise comparing the normalized read counts per amplicon and a baseline coverage for the associated exon to determine a candidate copy number for the exon. The step of determining exon copy numbers may further comprise applying a scaling procedure to the candidate copy numbers for amplicons generated from a germline sample. The scaling procedure may comprise (a) selecting the BRCA1 or BRCA2 gene having a maximum candidate copy number value; (b) determining a median value of the candidate copy number values for exons of the selected gene; (c) setting the median value to a reference level; and (d) scaling the candidate copy numbers of the exons of both genes relative to the reference level. The step of the determining exon copy numbers may further comprise applying a scaling procedure to the candidate copy numbers for amplicons generated from a somatic sample, wherein the scaling procedure may comprise: (a) determining a first median value of the candidate copy numbers for the exons of the BRCA1 gene and a second median value of the candidate copy numbers for the exons of the BRCA2 gene; (b) setting the first median value to a reference level; (c) scaling the candidate copy numbers for the exons of the BRCA1 gene relative to the reference level; (d) setting the second median value to the reference level; and (e) scaling the candidate copy numbers for the exons of the BRCA2 gene relative to the reference level. The step of detecting an exon deletion or an exon duplication may further comprise merging copy number calls for adjacent exons to form a copy number call for a segment of at least two exons. In the step of merging copy number calls, the candidate copy numbers for the adjacent exons may be within an interval of a same integer value. The step of detecting a whole gene deletion may further comprise calculating a first mean and a first standard deviation of the normalized read counts per amplicon associated with the BRCA1 gene and a second mean and a second standard deviation of the normalized read counts per amplicon associated with the BRCA2 gene. The step of detecting a whole gene deletion may further comprise applying a t-test to the first mean and the first standard deviation of the normalized read counts per amplicon associated with the BRCA1 gene and the second mean and the second standard deviation of the normalized read counts per amplicon associated with the BRCA2 gene. The step of applying a t-test may further comprise comparing a p-value to a first threshold to form a first comparison. The step of applying a t-test may further comprise calculating a PHRED score by multiplying (-10) times a log of the p-value. The step of detecting a whole gene deletion may further comprise (a) calculating a first ratio of the first standard deviation to the first mean and a second ratio of the second standard deviation to the second mean; (b) comparing the first ratio to a second threshold to form a second comparison; and (c) comparing the second ratio to the second threshold form a third comparison. The step of detecting a whole gene deletion may further comprise making a decision on a whole gene deletion event using results of the first, second and third comparisons. The method may further comprise detecting small variants in the BRCA1 and BRCA2 genes.

According to an exemplary embodiment not part of the invention, there is provided a kit comprising a set of primers associated with exons of BRCA1 and BRCA2 genes in a gene panel, the primers used in a method for detecting large rearrangements in the BRCA1 and BRCA2 genes, comprising: (a) amplifying a nucleic acid sample in the presence of a primer pool to produce a plurality of amplicons, the primer pool including a plurality of target specific primers targeting regions of exons of the BRCA1 and BRCA2 genes, wherein the target-specific primers for a region of an exon produce overlapping amplicons that cover the exon; (b) sequencing the amplicons to generate a plurality of reads; (c) mapping the reads to a reference sequence, wherein the reference sequence includes the BRCA1 and BRCA2 genes; (d) determining a number of reads per amplicon for the amplicons associated with the exons of the BRCA1 gene and a number of reads per amplicon for the amplicons associated with the exons of the BRCA2 gene; (e) determining exon copy numbers for the exons of the BRCA1 and BRCA2 genes based on the number of reads per amplicon for the amplicons associated with the exons of the BRCA1 and BRCA2 genes; (f) detecting an exon deletion or an exon duplication based on the exon copy numbers; and (g) detecting a whole gene deletion of BRCA1 gene or the BRCA2 gene based on the number of reads per amplicon for the amplicons associated with the exons of the BRCA1 and BRCA2 genes. The exons of the BRCA1 and BRCA2 genes may comprise coding exons. The method for use with the kit may further comprise dividing the number of reads per amplicon for amplicons associated with exons of the BRCA1 gene by a total number of reads of the amplicons generated from the nucleic acid sample to form normalized read counts per amplicon for the BRCA1 gene and dividing the number of reads per amplicon for amplicons associated with exons of the BRCA2 gene by a total number of reads of the amplicons generated from the nucleic acid sample to form normalized read counts per amplicon for the BRCA2 gene. The step of determining exon copy numbers may further comprise comparing the normalized read counts per amplicon and a baseline coverage for the associated exon to determine a candidate copy number for the exon. The step of determining exon copy numbers may further comprise applying a scaling procedure to the candidate copy numbers for amplicons generated from a germline sample. The scaling procedure may comprise (a) selecting the BRCA1 or BRCA2 gene having a maximum candidate copy number value; (b) determining a median value of the candidate copy number values for exons of the selected gene; (c) setting the median value to a reference level; and (d) scaling the candidate copy numbers of the exons of both genes relative to the reference level. The step of the determining exon copy numbers may further comprise applying a scaling procedure to the candidate copy numbers for amplicons generated from a somatic sample, wherein the scaling procedure may comprise: (a) determining a first median value of the candidate copy numbers for the exons of the BRCA1 gene and a second median value of the candidate copy numbers for the exons of the BRCA2 gene; (b) setting the first median value to a reference level; (c) scaling the candidate copy numbers for the exons of the BRCA1 gene relative to the reference level; (d) setting the second median value to the reference level; and (e) scaling the candidate copy numbers for the exons of the BRCA2 gene relative to the reference level. The step of detecting an exon deletion or an exon duplication may further comprise merging copy number calls for adjacent exons to form a copy number call for a segment of at least two exons. In the step of merging copy number calls, the candidate copy numbers for the adjacent exons may be within an interval of a same integer value. The step of detecting a whole gene deletion may further comprise calculating a first mean and a first standard deviation of the normalized read counts per amplicon associated with the BRCA1 gene and a second mean and a second standard deviation of the normalized read counts per amplicon associated with the BRCA2 gene. The step of detecting a whole gene deletion may further comprise applying a t-test to the first mean and the first standard deviation of the normalized read counts per amplicon associated with the BRCA1 gene and the second mean and the second standard deviation of the normalized read counts per amplicon associated with the BRCA2 gene. The step of applying a t-test may further comprise comparing a p-value to a first threshold to form a first comparison. The step of applying a t-test may further comprise calculating a PHRED score by multiplying (-10) times a log of the p-value. The step of detecting a whole gene deletion may further comprise (a) calculating a first ratio of the first standard deviation to the first mean and a second ratio of the second standard deviation to the second mean; (b) comparing the first ratio to a second threshold to form a second comparison; and (c) comparing the second ratio to the second threshold form a third comparison. The step of detecting a whole gene deletion may further comprise making a decision on a whole gene deletion event using results of the first, second and third comparisons. The method for use with the kit may further comprise detecting small variants in the BRCA1 and BRCA2 genes.

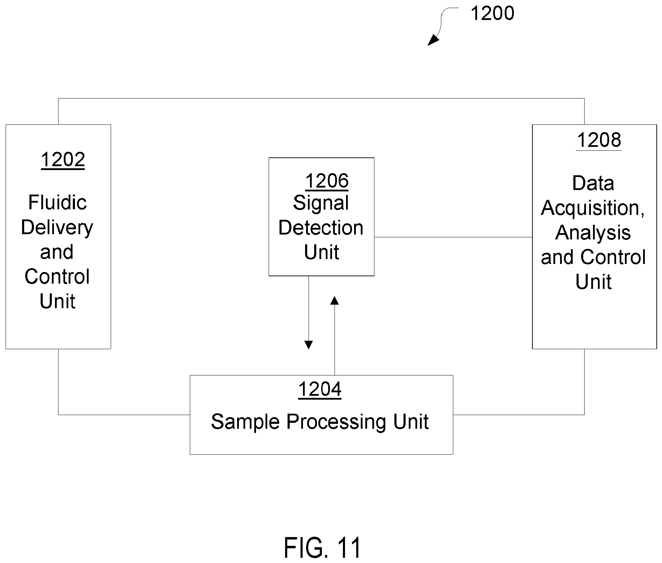

Various embodiments of nucleic acid sequencing platforms, such as a nucleic acid sequencer, can include components as displayed in the block diagram of FIG. 11. According to various embodiments, sequencing instrument 1200 can include a fluidic delivery and control unit 1202, a sample processing unit 1204, a signal detection unit 1206, and a data acquisition, analysis and control unit 1208. Various embodiments of instrumentation, reagents, libraries and methods used for next generation sequencing are described in U.S. Patent Application Publication No. 2009/0127589 and No. 2009/0026082. Various embodiments of instrument 1200 can provide for automated sequencing that can be used to gather sequence information from a plurality of sequences in parallel, such as substantially simultaneously.

In various embodiments, the fluidics delivery and control unit 1202 can include reagent delivery system. The reagent delivery system can include a reagent reservoir for the storage of various reagents. The reagents can include RNA-based primers, forward/reverse DNA primers, oligonucleotide mixtures for ligation sequencing, nucleotide mixtures for sequencing-by-synthesis, optional ECC oligonucleotide mixtures, buffers, wash reagents, blocking reagent, stripping reagents, and the like. Additionally, the reagent delivery system can include a pipetting system or a continuous flow system which connects the sample processing unit with the reagent reservoir.

In various embodiments, the sample processing unit 1204 can include a sample chamber, such as flow cell, a substrate, a micro-array, a multi-well tray, or the like. The sample processing unit 1204 can include multiple lanes, multiple channels, multiple wells, or other means of processing multiple sample sets substantially simultaneously. Additionally, the sample processing unit can include multiple sample chambers to enable processing of multiple runs simultaneously. In particular embodiments, the system can perform signal detection on one sample chamber while substantially simultaneously processing another sample chamber. Additionally, the sample processing unit can include an automation system for moving or manipulating the sample chamber.

In various embodiments, the signal detection unit 1206 can include an imaging or detection sensor. For example, the imaging or detection sensor can include a CCD, a CMOS, an ion or chemical sensor, such as an ion sensitive layer overlying a CMOS or FET, a current or voltage detector, or the like. The signal detection unit 1206 can include an excitation system to cause a probe, such as a fluorescent dye, to emit a signal. The excitation system can include an illumination source, such as arc lamp, a laser, a light emitting diode (LED), or the like. In particular embodiments, the signal detection unit 1206 can include optics for the transmission of light from an illumination source to the sample or from the sample to the imaging or detection sensor. Alternatively, the signal detection unit 1206 may provide for electronic or non-photon based methods for detection and consequently not include an illumination source. In various embodiments, electronic-based signal detection may occur when a detectable signal or species is produced during a sequencing reaction. For example, a signal can be produced by the interaction of a released byproduct or moiety, such as a released ion, such as a hydrogen ion, interacting with an ion or chemical sensitive layer. In other embodiments a detectable signal may arise as a result of an enzymatic cascade such as used in pyrosequencing (see, for example, U.S. Patent Application Publication No. 2009/0325145) where pyrophosphate is generated through base incorporation by a polymerase which further reacts with ATP sulfurylase to generate ATP in the presence of adenosine 5' phosphosulfate wherein the ATP generated may be consumed in a luciferase mediated reaction to generate a chemiluminescent signal. In another example, changes in an electrical current can be detected as a nucleic acid passes through a nanopore without the need for an illumination source.

In various embodiments, a data acquisition analysis and control unit 1208 can monitor various system parameters. The system parameters can include temperature of various portions of instrument 1200, such as sample processing unit or reagent reservoirs, volumes of various reagents, the status of various system subcomponents, such as a manipulator, a stepper motor, a pump, or the like, or any combination thereof.

It will be appreciated by one skilled in the art that various embodiments of instrument 1200 can be used to practice variety of sequencing methods including ligation-based methods, sequencing by synthesis, single molecule methods, nanopore sequencing, and other sequencing techniques.

In various embodiments, the sequencing instrument 1200 can determine the sequence of a nucleic acid, such as a polynucleotide or an oligonucleotide. The nucleic acid can include DNA or RNA, and can be single stranded, such as ssDNA and RNA, or double stranded, such as dsDNA or a RNA/cDNA pair. In various embodiments, the nucleic acid can include or be derived from a fragment library, a mate pair library, a ChIP fragment, or the like. In particular embodiments, the sequencing instrument 1200 can obtain the sequence information from a single nucleic acid molecule or from a group of substantially identical nucleic acid molecules.

In various embodiments, sequencing instrument 1200 can output nucleic acid sequencing read data in a variety of different output data file types/formats, including, but not limited to: *.fasta, *.csfasta, *seq.txt, *qseq.txt, *.fastq, *.sff, *prb.txt, *.sms, *srs and/or *.qv.

According to various exemplary embodiments, one or more features of any one or more of the above-discussed teachings and/or exemplary embodiments may be performed or implemented using appropriately configured and/or programmed hardware and/or software elements. Determining whether an embodiment is implemented using hardware and/or software elements may be based on any number of factors, such as desired computational rate, power levels, heat tolerances, processing cycle budget, input data rates, output data rates, memory resources, data bus speeds, etc., and other design or performance constraints.

Examples of hardware elements may include processors, microprocessors, input(s) and/or output(s) (I/O) device(s) (or peripherals) that are communicatively coupled via a local interface circuit, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth. The local interface may include, for example, one or more buses or other wired or wireless connections, controllers, buffers (caches), drivers, repeaters and receivers, etc., to allow appropriate communications between hardware components. A processor is a hardware device for executing software, particularly software stored in memory. The processor can be any custom made or commercially available processor, a central processing unit (CPU), an auxiliary processor among several processors associated with the computer, a semiconductor based microprocessor (e.g., in the form of a microchip or chip set), a macroprocessor, or generally any device for executing software instructions. A processor can also represent a distributed processing architecture. The I/O devices can include input devices, for example, a keyboard, a mouse, a scanner, a microphone, a touch screen, an interface for various medical devices and/or laboratory instruments, a bar code reader, a stylus, a laser reader, a radio-frequency device reader, etc. Furthermore, the I/O devices also can include output devices, for example, a printer, a bar code printer, a display, etc. Finally, the I/O devices further can include devices that communicate as both inputs and outputs, for example, a modulator/demodulator (modem; for accessing another device, system, or network), a radio frequency (RF) or other transceiver, a telephonic interface, a bridge, a router, etc.

Examples of software may include software components, programs, applications, computer programs, application programs, system programs, machine programs, operating system software, middleware, firmware, software modules, routines, subroutines, functions, methods, procedures, software interfaces, application program interfaces (API), instruction sets, computing code, computer code, code segments, computer code segments, words, values, symbols, or any combination thereof. A software in memory may include one or more separate programs, which may include ordered listings of executable instructions for implementing logical functions. The software in memory may include a system for identifying data streams in accordance with the present teachings and any suitable custom made or commercially available operating system (O/S), which may control the execution of other computer programs such as the system, and provides scheduling, input-output control, file and data management, memory management, communication control, etc.

According to various exemplary embodiments, one or more features of any one or more of the above-discussed teachings and/or exemplary embodiments may be performed or implemented using appropriately configured and/or programmed non-transitory machine-readable medium or article that may store an instruction or a set of instructions that, if executed by a machine, may cause the machine to perform a method and/or operations in accordance with the exemplary embodiments. Such a machine may include, for example, any suitable processing platform, computing platform, computing device, processing device, computing system, processing system, computer, processor, scientific or laboratory instrument, etc., and may be implemented using any suitable combination of hardware and/or software. The machine-readable medium or article may include, for example, any suitable type of memory unit, memory device, memory article, memory medium, storage device, storage article, storage medium and/or storage unit, for example, memory, removable or non-removable media, erasable or non-erasable media, writeable or re-writeable media, digital or analog media, hard disk, floppy disk, read-only memory compact disc (CD-ROM), recordable compact disc (CD-R), rewriteable compact disc (CD-RW), optical disk, magnetic media, magneto-optical media, removable memory cards or disks, various types of Digital Versatile Disc (DVD), a tape, a cassette, etc., including any medium suitable for use in a computer. Memory can include any one or a combination of volatile memory elements (e.g., random access memory (RAM, such as DRAM, SRAM, SDRAM, etc.)) and nonvolatile memory elements (e.g., ROM, EPROM, EEROM, Flash memory, hard drive, tape, CDROM, etc.). Moreover, memory can incorporate electronic, magnetic, optical, and/or other types of storage media. Memory can have a distributed architecture where various components are situated remote from one another, but are still accessed by the processor. The instructions may include any suitable type of code, such as source code, compiled code, interpreted code, executable code, static code, dynamic code, encrypted code, etc., implemented using any suitable high-level, low-level, object-oriented, visual, compiled and/or interpreted programming language.

According to various exemplary embodiments, one or more features of any one or more of the above-discussed teachings and/or exemplary embodiments may be performed or implemented at least partly using a distributed, clustered, remote, or cloud computing resource.

According to various exemplary embodiments, one or more features of any one or more of the above-discussed teachings and/or exemplary embodiments may be performed or implemented using a source program, executable program (object code), script, or any other entity comprising a set of instructions to be performed. When a source program, the program can be translated via a compiler, assembler, interpreter, etc., which may or may not be included within the memory, so as to operate properly in connection with the O/S. The instructions may be written using (a) an object oriented programming language, which has classes of data and methods, or (b) a procedural programming language, which has routines, subroutines, and/or functions, which may include, for example, C, C++, R, Pascal, Basic, Fortran, Cobol, Perl, Java, and Ada.

According to various exemplary embodiments, one or more of the above-discussed exemplary embodiments may include transmitting, displaying, storing, printing or outputting to a user interface device, a computer readable storage medium, a local computer system or a remote computer system, information related to any information, signal, data, and/or intermediate or final results that may have been generated, accessed, or used by such exemplary embodiments. Such transmitted, displayed, stored, printed or outputted information can take the form of searchable and/or filterable lists of runs and reports, pictures, tables, charts, graphs, spreadsheets, correlations, sequences, and combinations thereof, for example.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention.

## Claims

1. A method for detecting large rearrangements in BRCA1 and BRCA2 genes, comprising:

   amplifying a nucleic acid sample in the presence of a primer pool to produce a plurality of amplicons, the primer pool including a plurality of target specific primers targeting regions exons of the BRCA1 and BRCA2 genes, wherein the target-specific primers for a region of an exon produce overlapping amplicons that cover the exon;
   sequencing the amplicons to generate a plurality of reads;
   mapping the reads to a reference sequence, wherein the reference sequence includes the BRCA1 and BRCA2 genes;
   determining a number of reads per amplicon for the amplicons associated with the exons of the BRCA1 gene and a number of reads per amplicon for the amplicons associated with the exons of the BRCA2 gene;
   dividing the number of reads per amplicon for amplicons associated with exons of the BRCA1 gene by a total number of reads of the amplicons generated from the nucleic acid sample to form normalized read counts per amplicon for the BRCA1 gene;
   dividing the number of reads per amplicon for amplicons associated with exons of the BRCA2 gene by a total number of reads of the amplicons generated from the nucleic acid sample to form normalized read counts per amplicon for the BRCA2 gene;
   determining exon copy numbers for the exons of the BRCA1 and BRCA2 genes based on the number of reads per amplicon for the amplicons associated with the exons of the BRCA1 and BRCA2 genes;

wherein the determining exon copy numbers further comprises comparing the normalized read counts per amplicon and a baseline coverage for the associated exon to determine a candidate copy number for the exon;

applying a scaling procedure to the candidate copy numbers for amplicons generated from a germline sample, the scaling procedure comprising: selecting the BRCA1 or BRCA2 gene having a maximum candidate copy number value;

determining a median value of the candidate copy number values for exons of the selected gene; setting the median value to a reference level;

scaling the candidate copy numbers of the exons of both genes relative to the reference level;

detecting an exon deletion or an exon duplication based on the exon copy numbers; and

detecting a whole gene deletion of the BRCA1 gene or the BRCA2 gene based on the number of reads per amplicon for the amplicons associated with the exons of the BRCA1 and BRCA2 genes.

**2.** The method of claim 1, wherein the exons of the BRCA1 and BRCA2 genes comprise coding exons.

**3.** The method of claim 1, wherein the determining exon copy numbers further comprises applying a scaling procedure to the candidate copy numbers for amplicons generated from a somatic sample, the scaling procedure comprising: determining a first median value of the candidate copy numbers for the exons of the BRCA1 gene and a second median value of the candidate copy numbers for the exons of the BRCA2 gene;

setting the first median value to a reference level;

scaling the candidate copy numbers for the exons of the BRCA1 gene relative to the reference level;

setting the second median value to the reference level; and

scaling the candidate copy numbers for the exons of the BRCA2 gene relative to the reference level.

**4.** The method of claim 1, wherein the detecting an exon deletion or an exon duplication further comprises merging copy number calls for adjacent exons to form a copy number call for a segment of at least two exons.

**5.** The method of claim 4, wherein the candidate copy numbers for the adjacent exons are within an interval of a same integer value.

**6.** The method of claim 1, wherein the detecting a whole gene deletion further comprises calculating a first mean and a first standard deviation of the normalized read counts per amplicon associated with the BRCA1 gene and a second mean and a second standard deviation of the normalized read counts per amplicon associated with the BRCA2 gene.

**7.** The method of claim 6, wherein the detecting a whole gene deletion further comprises applying a t-test to the first mean and the first standard deviation of the normalized read counts per amplicon associated with the BRCA1 gene and the

second mean and the second standard deviation of the normalized read counts per amplicon associated with the BRCA2 gene.

**8.** The method of claim 7, wherein the applying a t-test further comprises comparing a p-value to a first threshold to form a first comparison.

**9.** The method of claim 8, wherein the applying a t-test further comprises calculating a PHRED score by multiplying -10 times a log of the p-value.

**10.** The method of claim 9, wherein the detecting a whole gene deletion further comprises

calculating a first ratio of the first standard deviation to the first mean and a second ratio of the second standard deviation to the second mean;

comparing the first ratio to a second threshold to form a second comparison; and

comparing the second ratio to the second threshold form a third comparison.

**11.** The method of claim 10, wherein the detecting a whole gene deletion further comprises making a decision on a whole gene deletion event using results of the first, second and third comparisons.

**12.** The method of claim 1, further comprising detecting small variants in the BRCA1 and BRCA2 genes.

**Patentansprüche**

**1.** Verfahren zum Erkennen großer Umlagerungen im BRCA1- und BRCA2-Gen, umfassend:

Amplifizieren einer Nukleinsäureprobe in Gegenwart eines Primerpools, um eine Vielzahl von Amplikonen zu erzeugen, wobei der Primerpool eine Vielzahl zielspezifischer Primer einschließt, die auf Exonregionen der BRCA1- und BRCA2-Gene zielen, wobei die zielspezifischen Primer für eine Region eines Exons überlappende Amplikone erzeugen, die das Exon abdecken;
Sequenzieren der Amplikone, um eine Vielzahl von Reads zu generieren;
Zuordnen der Reads zu einer Referenzsequenz, wobei die Referenzsequenz die BRCA1- und BRCA2-Gene einschließt;
Bestimmen einer Anzahl von Reads pro Amplikon für die Amplikone, die mit den Exonen des BRCA1-Gens assoziiert sind und einer Anzahl von Reads pro Amplikon für die Amplikone, die mit den Exonen des BRCA2-Gens assoziiert sind;
Teilen der Anzahl der Reads pro Amplikon für Amplikone, die mit Exonen des BRCA1-Gens assoziiert sind, durch die Gesamtzahl der Reads der aus der Nukleinsäureprobe generierten Amplikone, um normalisierte Read-Zählungen pro Amplikon für das BRCA1-Gen zu bilden;
Teilen der Anzahl der Reads pro Amplikon für Amplikone, die mit Exonen des BRCA2-Gens assoziiert sind, durch die Gesamtzahl der Reads der aus der Nukleinsäureprobe generierten Amplikone, um normalisierte Read-Zahlen pro Amplikon für das BRCA2-Gen zu bilden;
Bestimmen der Exonkopienzahlen für die Exone der BRCA1- und BRCA2-Gene basierend auf der Anzahl der Reads pro Amplikon für die Amplikone, die mit den Exonen der BRCA1- und BRCA2-Gene assoziiert sind;
wobei das Bestimmen der Exonkopienzahlen ferner das Vergleichen der normalisierten Read-Zahlen pro Amplikon und einer Basisabdeckung für das assoziierte Exon umfasst, um eine Kandidatenkopienzahl für das Exon zu bestimmen;
Anwenden einer Skalierungsprozedur auf die Kandidatenkopienzahlen für aus einer Keimbahnprobe erzeugte Amplikone, wobei die Skalierungsprozedur umfasst: Auswählen des BRCA1- oder BRCA2-Gens mit einem maximalen Kandidatenkopienzahlwert;
Bestimmen eines Medianwerts der Kandidatenkopienzahlwerte für Exone des ausgewählten Gens; Festlegen des Medianwerts auf ein Referenzniveau;
Skalieren der Kandidatenkopienzahlen der Exone beider Gene im Verhältnis zum Referenzniveau;
Erkennen einer Exondeletion oder einer Exonduplikation basierend auf der Exonkopienzahl; und
Erkennen einer vollständigen Gendeletion des BRCA1-Gens oder des BRCA2-Gens basierend auf der Anzahl der Reads pro Amplikon für die Amplikone, die mit den Exonen des BRCA1- und des BRCA2-Gens assoziiert sind.

**2.** Verfahren nach Anspruch 1, wobei die Exone der BRCA1- und BRCA2-Gene codierende Exone umfassen.

**3.** Verfahren nach Anspruch 1, wobei das Bestimmen der Exonkopienzahlen ferner das Anwenden einer Skalierungsprozedur auf die Kandidatenkopienzahlen für aus einer somatischen Probe erzeugte Amplikone umfasst, wobei die Skalierungsprozedur umfasst: Bestimmen eines ersten Medianwerts der Kandidatenkopienzahlen für die Exone des BRCA1-Gens und eines zweiten Medianwerts der Kandidatenkopienzahlen für die Exone des BRCA2-Gens;

Festlegen des ersten Medianwerts auf ein Referenzniveau;
Skalieren der Kandidatenkopienzahlen für die Exone des BRCA1-Gens im Verhältnis zum Referenzniveau;
Festlegen des zweiten Medianwerts auf das Referenzniveau; und
Skalieren der Kandidatenkopienzahlen für die Exone des BRCA2-Gens im Verhältnis zum Referenzniveau.

**4.** Verfahren nach Anspruch 1, wobei das Erkennen einer Exondeletion oder einer Exonduplikation ferner das Zusammenführen von Kopienzahlaufrufen für benachbarte Exone umfasst, um einen Kopienzahlaufruf für ein Segment von mindestens zwei Exonen zu bilden.

**5.** Verfahren nach Anspruch 4, wobei die Kandidatenkopienzahlen für die benachbarten Exone innerhalb eines Intervalls eines gleichen ganzzahligen Werts liegen.

**EP 3 631 018 B1**

**6.** Verfahren nach Anspruch 1, wobei das Erkennen einer Deletion eines gesamten Gens ferner das Berechnen eines ersten Mittelwerts und einer ersten Standardabweichung der normalisierten Read-Zahlen pro Amplikon, die mit dem BRCA1-Gen assoziiert sind, und eines zweiten Mittelwerts und einer zweiten Standardabweichung der normalisierten Read-Zahlen pro Amplikon, die mit dem BRCA2-Gen assoziiert sind, umfasst.

**7.** Verfahren nach Anspruch 6, wobei das Erkennen einer Deletion eines gesamten Gens ferner das Anwenden eines t-Tests auf den ersten Mittelwert und die erste Standardabweichung der normalisierten Read-Zahlen pro Amplikon, die mit dem BRCA1-Gen assoziiert sind, und den zweiten Mittelwert und die zweite Standardabweichung der normalisierten Read-Zahlen pro Amplikon, die mit dem BRCA2-Gen assoziiert sind, umfasst.

**8.** Verfahren nach Anspruch 7, wobei das Anwenden eines t-Tests ferner umfasst:
Vergleichen eines p-Werts mit einem ersten Schwellenwert, um einen ersten Vergleich zu bilden.

**9.** Verfahren nach Anspruch 8, wobei das Anwenden eines t-Tests ferner umfasst:
Berechnen eines PHRED-Scores durch Multiplikation von -10 mit einem Logarithmus des p-Werts.

**10.** Verfahren nach Anspruch 9, wobei das Erkennen einer Deletion eines gesamten Gens ferner das Berechnen eines ersten Verhältnisses der ersten Standardabweichung mit dem ersten Mittelwert und eines zweiten Verhältnisses der zweiten Standardabweichung mit dem zweiten Mittelwert umfasst;

Vergleichen des ersten Verhältnisses mit einem zweiten Schwellenwert, um einen zweiten Vergleich zu bilden; und
Vergleichen des zweiten Verhältnisses mit dem zweiten Schwellenwert, um einen dritten Vergleich zu bilden.

**11.** Verfahren nach Anspruch 10, wobei das Erkennen einer Deletion eines gesamten Gens ferner das Treffen einer Entscheidung über ein Ereignis einer Deletion eines gesamten Gens unter Verwendung der Ergebnisse des ersten, zweiten und dritten Vergleichs umfasst.

**12.** Verfahren nach Anspruch 1, ferner umfassend das Erkennen kleiner Varianten in dem BRCA1- und dem BRCA2-Gen.

**Revendications**

**1.** Procédé de détection de grands réarrangements dans les gènes BRCA1 et BRCA2, comprenant :

l'amplification d'un échantillon d'acide nucléique en présence d'un groupe d'amorces pour produire une pluralité d'amplicons, le groupe d'amorces comportant une pluralité d'amorces spécifiques ciblant des régions exons des gènes BRCA1 et BRCA2, dans lequel les amorces spécifiques ciblent une région d'un exon produisant des amplicons qui se chevauchent et qui couvrent l'exon ;
le séquençage des amplicons pour générer une pluralité de lectures ;
la mise en correspondance des lectures avec une séquence de référence, dans lequel la séquence de référence comporte les gènes BRCA1 et BRCA2 ;
la détermination d'un nombre de lectures par amplicon pour les amplicons associés aux exons du gène BRCA1 et d'un nombre de lectures par amplicon pour les amplicons associés aux exons du gène BRCA2 ;
la division du nombre de lectures par amplicon pour les amplicons associés aux exons du gène BRCA1 par le nombre total de lectures des amplicons générés à partir de l'échantillon d'acide nucléique pour former des nombres de lectures normalisés par amplicon pour le gène BRCA1 ;
la division du nombre de lectures par amplicon pour les amplicons associés aux exons du gène BRCA2 par le nombre total de lectures des amplicons générés à partir de l'échantillon d'acide nucléique pour former des nombres de lectures normalisés par amplicon pour le gène BRCA2 ;
la détermination du nombre de copies d'exon pour les exons des gènes BRCA1 et BRCA2 sur la base du nombre de lectures par amplicon pour les amplicons associés aux exons des gènes BRCA1 et BRCA2 ;
la détermination du nombre de copies de l'exon consiste en outre à comparer le nombre de lectures normalisé par amplicon et une couverture de base pour l'exon associé afin de déterminer un nombre de copies candidat pour l'exon ;
l'application d'une procédure de mise à l'échelle aux nombres de copies candidats pour les amplicons générés à partir d'un échantillon germinal, la procédure de mise à l'échelle comprenant : la sélection du gène BRCA1 ou BRCA2 ayant une valeur maximale de nombre de copies candidat ;
la détermination d'une valeur médiane des valeurs de nombre de copies candidates pour les exons du gène sélectionné ; le réglage de la valeur médiane à un niveau de référence ;
la mise à l'échelle du nombre de copies candidates des exons des deux gènes par rapport au niveau de référence ;
la détection d'une suppression ou d'une duplication d'exon sur la base des nombres de copies d'exon ; et
la détection d'une délétion du gène BRCA1 ou du gène BRCA2 sur la base du nombre de lectures par amplicon pour les amplicons associés aux exons des gènes BRCA1 et BRCA2.

2. Procédé selon la revendication 1, dans lequel les exons des gènes BRCA1 et BRCA2 comprennent des exons codants.

3. Procédé selon la revendication 1, dans lequel la détermination des nombres de copies d'exon comprend en outre l'application d'une procédure de mise à l'échelle aux nombres de copies candidats pour les amplicons générés à partir d'un échantillon somatique, la procédure de mise à l'échelle comprenant : la détermination d'une première valeur médiane des nombres de copies candidats pour les exons du gène BRCA1 et d'une seconde valeur médiane des nombres de copies candidats pour les exons du gène BRCA2 ;

le réglage de la première valeur médiane à un niveau de référence ;
la mise à l'échelle du nombre de copies candidates pour les exons du gène BRCA1 par rapport au niveau de référence ;
le réglage de la seconde valeur médiane au niveau de référence ; et
la mise à l'échelle du nombre de copies candidates pour les exons du gène BRCA2 par rapport au niveau de référence.

4. Procédé selon la revendication 1, dans lequel la détection d'une délétion d'exon ou d'une duplication d'exon comprend en outre la fusion des appels de numéro de copie pour les exons adjacents afin de former un appel de numéro de copie pour un segment d'au moins deux exons.

5. Procédé selon la revendication 4, dans lequel les nombres de copies candidats pour les exons adjacents se situent dans un intervalle d'une même valeur entière.

6. Procédé selon la revendication 1, dans lequel la détection d'une délétion du gène entier comprend en outre le calcul d'une première moyenne et d'un premier écart-type du nombre de lectures normalisées par amplicon associé au gène BRCA1 et d'une seconde moyenne et d'un second écart-type du nombre de lectures normalisées par amplicon associé au gène BRCA2.

7. Procédé selon la revendication 6, dans laquelle la détection d'une délétion du gène entier comprend en outre l'application d'un test t à la première moyenne et au premier écart-type des nombres normalisés de lectures par amplicon associés au gène BRCA1 et la
seconde moyenne et le second écart type des nombres de lectures normalisés par amplicon associé au gène BRCA2.

8. Procédé selon la revendication 7, dans lequel l'application d'un test t comprend en outre
la comparaison d'une valeur p à un premier seuil pour former une première comparaison.

9. Procédé selon la revendication 8, dans lequel l'application d'un test t comprend en outre
le calcul d'un score PHRED en multipliant par -10 le logarithme de la valeur p.

10. Procédé selon la revendication 9, dans lequel la détection d'une délétion de gène entier comprend en outre le calcul d'un premier rapport entre le premier écart type et la première moyenne et d'un second rapport entre le second écart type et la seconde moyenne ;

la comparaison du premier rapport à un second seuil pour former une deuxième comparaison ; et
la comparaison du second rapport au second seuil constitue une troisième comparaison.

11. Procédé selon la revendication 10, dans lequel la détection d'une délétion de gène entier consiste en outre à prendre une décision sur un événement de délétion de gène entier à l'aide des résultats des première, deuxième et troisième comparaisons.

12. Procédé selon la revendication 1, comprenant en outre la détection de petits variants dans les gènes BRCA1 et BRCA2.

FIG. 1

FIG. 2

SIGNAL
MEASUREMENTS

⭣

BASE CALLER — 302

⭣

MAPPER — 304

⭣

GENERATE READ COUNTS — 306

308 ⌐                          ⌐ 310

SMALL VARIANT CALLER          LARGE REARRANGEMENT
                              DETECTOR

⭣                              ⭣

DISPLAY — 312

FIG. 3

310

402
GENE DELETION CALLER

404
EXON COPY NUMBER CALLER

406
CNV EVALUATOR

FIG. 4

FIG. 5

FIG. 6

FIG. 7

FIG. 8

FIG. 9

FIG. 10

EP 3 631 018 B1

1200

| 1202<br><br>Fluidic<br>Delivery<br>and<br>Control<br>Unit | | 1206<br>Signal<br>Detection<br>Unit | | 1208<br><br>Data<br>Acquisition,<br>Analysis<br>and Control<br>Unit |

1204

Sample Processing Unit

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120295819
- US 20130090860 A
- US 20120197623 A
- US 20130345066 A
- US 20140296080 A
- US 20140052381 A
- US 20140256571 A
- US 20090127589
- US 20090026082 A
- US 20090325145

### Non-patent literature cited in the description

- **HIROTSU et al.** *Oncotarget,* 2017, vol. 8 (70), 114463-114473